# EUROPEAN PATENT SPECIFICATION

(11) **EP 0 557 625 B1**
(45) Date of publication and mention of the grant of the patent: **06.10.1999**
(21) Application number: 92300575.5
(22) Date of filing: 23.01.1992
(51) Int. Cl.: B29C 41/14, A61F 6/04, A61L 31/00

(54) **Method of making latex articles**
Verfahren zum Herstellen von Latexgegenständen
Procédé pour la fabrication d'articles en latex

(43) Date of publication of application: 01.09.1993
(60) Divisional application: 99103415.8
(73) Proprietor: Beck, Robin Thill, Los Angeles, California 90049 (DE)
(72) Inventor: Beck, Robin Thill, Los Angeles, California 90049 (US); Solomons, Clive C., Denver, CO 80222 (US); Plunkett, Jerry D., Denver, Colorado 80210 (US); Smith, Clayton S., Golden, Colorado 80401 (US)
(74) Representative: Grünecker, Kinkeldey, Stockmair & Schwanhäusser Anwaltssozietät

(56) References cited:
- EP-A- 0 141 628
- EP-A- 0 306 389
- EP-A- 0 368 456
- EP-A- 0 427 997
- WO-A-89/04647
- WO-A-90/01956
- DE-C- 442 551
- FR-A- 2 663 035
- DATABASE WPIL Week 9230, Derwent Publications Ltd., London, GB; AN 92-249398 & US-A-5 128 168 (J.D. PLUNKETT ET AL) 7 July 1992

## Description

This invention relates to the manufacture of latex articles incorporating a biocidal material for preventing the transmission and dissemination of disease and is especially applicable to the manufacture of such articles as gloves and condoms.

Latex has long been used for the manufacture of gloves and condoms for the purpose of inhibiting the transmission of disease-producing microbes and other harmful agents. Compare FR-A-2 663 035. Both the chemical inertness and the physical density of cured latex make it difficult for molecules and microbes to pass through the structure of the latex material. Nevertheless, latex materials are known to possess imperfections in the form of pits, pores and holes which can facilitate the transmission of such microbes and harmful agents through the latex material.

The present invention seeks to provide a procedure by means of which it is possible to make a chemical barrier against the transmission of such microbes and other harmful agents through a membrane such as latex.

The present invention now provides a method of forming a chemical barrier against the transmission of disease-causing microbes and other harmful agents through a membrane such as latex. In the method of the invention, a mold or former is coated with a biocide/coagulant which is dried, then the former is dipped into liquid latex. In an alternative procedure, the former is initially dipped into liquid latex which is allowed to gel before being dipped into the biocide/coagulant. The entire coating on the former is then cured. Alternatively, the biocide may be sprayed or otherwise applied onto the former or the gelled latex.

The present invention concerns the forming of a chemical barrier against disease-causing microbes and other harmful agents through a membrane fashioned from natural latex or from a synthetic latex material such as those known as natural skin, solvent cast membranes, elastomers, and polymers formed by curing the material from a liquid state. For convenience, the preferred aspects of the invention will be described with reference to a latex material. The latex material may be fashioned as a glove, condom, diaphragm, slipper, overshoe, sterile band, catheter, tubing, diaphragm, drape, gut opening, mouthpiece, baby nipple, intragastric nasal tube, nasal gastric tube, kidney shunt, rubber dam for teeth, plastic brace for teeth, sub-clavian vein or artery shunt, colostomy bag or any other product. Normally such latex products will be adapted for use in juxtaposition to a person's or animal's skin.

As used in the context of the present application, the term "biocide" means a substance or material which renders the disease-producing characteristic or harm-causing characteristic of a microbe or harmful agent ineffective substantially upon contact or shortly after contact of the microbe or harmful agent with the biocide. Examples of suitable biocides are dextran sulphate, nonoxynol-9, benzalkonium, betadyne, gentian violet, acriflavine and acridine dyes, mercurochrome, silver salts and an extract of blue green algae. Other examples of suitable biocides are set out in the list below.

Latex products, such as latex gloves and condoms, are conventionally formed by either a single dip or a double dip process. In these processes, a mold or former in the shape of the desired latex product is initially covered with a chemical coagulant, which allows a relatively thick, uniform, continuous layer of latex to be deposited on the former. The coagulant is dried on the former, and then the former is dipped into a vat of liquid latex for an appropriate period of time, whereby a film of latex is picked up on the surface of the former. The latex is allowed to wet gel into a sticky or tacky state and while in the gel state, is leached in a warm water rinse or bath. The warm water removes the coagulant as well as any residual ammonia or potassium hydroxide which might be present. In order to increase the thickness of latex, the process may be repeated so that a second film of latex is built up integrally over the first layer of latex. Usually the second layer is less thick than the first layer.

In accordance with the invention, the coagulant is selected so as to have biocidal properties so that the final latex product incorporates a biocide barrier. The method of the invention permits such latex products to be made by the general procedure described above and by the alternative procedure of initially applying a first liquid latex coating to the mold or former and then applying the biocide/coagulant to that first liquid latex coating before applying one or more further liquid latex coatings over the biocide/coagulant.

A biocide barrier can be incorporated into a latex product in the above manner, as follows. After the first dip of the former into the liquid latex and after the preferred warm water leach in a conventional manner, the former with the liquid latex in a wet gel state is dipped into a 0.10 to 5% by weight solution of gentian violet, for example a 0.33% by weight solution in water. The biocide solution coats the liquid latex. Alternatively, the biocide solution may be sprayed or otherwise applied to the wet gel latex. The biocide solution coating is then dried. Thereafter, the former is dipped again into a vat of liquid latex, may be leached again, and then the entire coating on the former is cured by drying.

It appears that the biocide solution acts as a coagulant, and causes the second layer of latex to be relatively thicker than is normally achieved without any biocide solution.

The amount of biocide solution applied either to the former or to the initial latex coating on the former is preferably such that the weight ratio of that biocide solution coating to the subsequent latex coating is 0.05 to 0.3.

A solution of 1.5% by weight of for example, gentian violet in water has also been used in this process, with slightly less desirable uniformity in thickness of the coatings. Nevertheless, it is believed that concentrations of up to about 5% by weight of gentian violet in water or in water and alcohol with or without other cosolvents can be advantageously used in the process.

It has been observed that the maximum amount of biocide solution picked-up by the wet gel latex coating the former is about 0.2 grams of solution per gram of latex.

It is believed that the biocide is bonded to and is permanently diffused within the surface of the latex, thereby substantially filling the pores and other imperfections of the latex.

The following biocides are believed to be equally suitable to those described above for use in the invention:
halogen and halogen compounds - chlorine and iodine;
phenolic compounds;
alcohols;
oxidants;
chlorhexidine;
nitrogen compounds;
surface active agents, such as
   quaternary ammonium compounds,
   acid anionic compounds,
   amphoteric compounds,
   heavy metals and
   dyes, e.g. crystal violet;
antibiotics;
HC BLUE NO. 2
   (N¹,N⁴,N⁴,-(2-hydroxyethyl)-2-nitro-p-phenylene-diamine);
NONOXYNOL-2
   (polyoxyethylene (2) nonyl phenyl ether); NONOXYNOL-4 and -8;
D & C RED NO. 22
   (eosine YS);
D & C RED NO. 37
   (rhodamine B-stearate);
D & C RED NO. 37 CALCIUM LAKE
   (rhodamine B stearate solvent);
and the following products listed according to their recommended or generally accepted biocidal applications:-
Antimicrobial Soaps:
   Cloflucarban
   Para-chloro-mein-xylenof
   Povidone-iodine complex
   1.5 percent phenol or less aqueous/alcoholic
   Triclocarbon
   Tricloean
Health-care Peronnel Handwash:
   Benzalkonium chloride
   Benzethonium chloride
   Clofluearban
   Hexylesorinal
   Iodine complexed with phophate eater of alkyaryloxy polyethlene glycol
   Methyl-benzethonium chloride
   Nonyl phenoxypoly (ethyleneoxy) ethanol-iodine
   Para-chloro-meta-xylenol
   Povidene-iodine complex
   1.5 percent phenol or less aqueous/alcoholic
   Poloxamer-iodine complex
   Tricloearban¹
   Undecoylium chloride-iodine complex
Patient preoperative skin preparation
   Bonzalkonium chloride
   Benzethonium chloride
   Hexylresorcinoi
   Iodine complexed with phosphate ester of alkylaryloxy polyethylene glycol
   Methylbenxethonium chloride
   Nonyl phenoxypoly (ethyleneoxy) ethanoilodine
   Para-thloro-meta-xylenol
   1.5 percent phenol or less aqueous/alcoholic
   Poloxamer-iodine complex
   Povidene-iodine complex
   Undecoylium chloride-iodine complex
Skin antiseptic
   Benzalkonium chloride
   Benzathonium chloride
   Hexylresorcinoi
   Iodine complexed with phosphate ester of alkylaryloxy polyethylene glycol
   Iodine tincture
   Methyl-bonzethonium chloride
   Nonyl phenoxypoly (ethylencoxy) ethanoliodine
   Para-chloro-meta-xylenol
   1.5 percent phenol or less aqueous/alcoholic
   Poloxamer-iodine complex
   Povidene-iodine complex
   Triclosan
   Triple Dye
   Undecoylium chloride-iodine complex
Skin wound cleanser
   Cloflutarban¹
   Iodine complexed with phosphate ester of alkylaryloxy
   polyethylene glycol
   Iodine tincture
   Nonyl phenoxypoly (ethyleneoxy) ethanoliodine
   Para-chloro-meta-xylenol
   1.5 percent phenol or less aqueous/alcoholic
   Poloxamer-iodine complex
   Povidene-iodine complex
   Tricloearban¹
   Triclosan
   Undecoylium chloride-iodine complex
Skin wound protectant
   Benzalkonium chloride
   Benzathonium chloride
   Hexylresorcinoi
   Iodine complexed with phosphate ester of alkylaryloxy
   polyethylene glycol
   Iodine tincture
   Methyl-bonzethonium chloride
   Nonyl phenoxypoly (ethylencoxy) ethanoliodine
   Para-chloro-meta-xylenol

## Claims

1. A method of making a latex article having a biocide barrier, comprising the steps of:
- applying onto a former a first coating consisting essentially of liquid latex and free of biocide;
- applying to the first coating of liquid latex a second coating consisting essentially of a biocide effective as a coagulant for liquid latex; and
- applying to said second coating a third coating consisting essentially of liquid latex and free of biocide.

2. The method according to claim 1, wherein the third coating is thicker than the first coating.

3. The method according to claim 1 or 2, wherein the first coating is leached with water prior to application of the second coating.

4. The method according to any of claims 1 to 3, wherein the second coating is dried before applying the third coating.

5. The method according to any of claims 1 to 4, wherein said first coating is applied by dipping the former into liquid latex.

6. The method according to any of claims 1 to 5, wherein the second coating is applied onto the first coating when the latex in the first coating is in a wet gel state.

7. The method according to any of claims 1 to 6, wherein the second coating is applied by spraying a biocide solution.

8. The method according to any of claims 1 to 6, wherein said second coating is applied by dipping the former into a biocide solution.

9. The method according to any of claims 1 to 8, wherein the biocide is applied as a solution having a concentration of 0.10 to 5 percent by weight.

10. The method according to any of claims 1 to 9, wherein said third coating is applied by dipping the former in a vat of liquid latex.

11. The method according to any of claims 1 to 10, wherein the biocide is selected from chlorhexidine, dextran sulfate, triclosan, benzalkonium, betadyne, gentian violet, acriflavine and acridine dyes, mercurochrome, silver salts, and an extract of blue-green algae.

12. The method according to any of claims 1 to 11, wherein the weight ration of the biocide solution to the first latex coating is from 0.05 to 0.3.

13. The method according to any of claims 1 to 12, wherein the weight ratio of the biocide solution in the second coating to the latex applied in the third coating is between 0.05 and 0.3.

14. The method according to any of claims 1 to 13, wherein the biocide is bonded to and is permanently diffused within the surface of the latex membrane.

## Patentansprüche

1. Verfahren zur Herstellung eines Latexartikels mit einer Biozidbarierre, umfassend die folgenden Schritte:
- Aufbringen einer ersten Schicht, die im wesentlichen aus flüssigem Latex besteht und frei von Bioziden ist, auf eine Form;
- Aufbringen einer zweiten Schicht, die im wesentlichen aus einem Biozid besteht, das als Koagulant für flüssigen Latex wirksam ist, auf die erste Schicht aus flüssigem Latex; und
- Aufbringen einer dritten Schicht, die im wesentlichen aus flüssigem Latex besteht und frei von Bioziden ist, auf diese zweite Schicht.

2. Verfahren nach Anspruch 1, worin die dritte Schicht dicker ist als die erste Schicht.

3. Verfahren nach Anspruch 1 oder 2, worin die erste Schicht mit Wasser ausgelaugt wird, bevor die zweite Schicht aufgebracht wird.

4. Verfahren nach einem der Ansprüche 1 bis 3, worin die zweite Schicht getrocknet wird, bevor die dritte Schicht aufgebracht wird.

5. Verfahren nach einem der Ansprüche 1 bis 4, worin die erste Schicht durch Eintauchen der Form in flüssigen Latex aufgebracht wird.

6. Verfahren nach einem der Ansprüche 1 bis 5, worin die zweite Schicht auf die erste Schicht aufgebracht wird, wenn der Latex der ersten Schicht den Zustand eines nassen Gels hat.

7. Verfahren nach einem der Ansprüche 1 bis 6, worin die zweite Schicht durch Aufsprühen einer Biozidlösung aufgebracht wird.

8. Verfahren nach einem der Ansprüche 1 bis 6, worin die zweite Schicht durch Eintauchen der Form in eine Biozidlösung aufgebracht wird.

9. Verfahren nach einem der Ansprüche 1 bis 8, worin das Biozid als Lösung mit einer Konzentration von 0,10 bis 5 Gew.-% aufgebracht wird.

10. Verfahren nach einem der Ansprüche 1 bis 9, worin die dritte Schicht durch Eintauchen der Form in einen Bottich mit flüssigem Latex aufgebracht wird.

11. Verfahren nach einem der Ansprüche 1 bis 10, worin das Biozid gewählt wird aus Chlorhexidin, Dextransulfat, Triclosan, Benzalkonium, Betadyne, Gentianaviolett, Acriflavin und Acridin-Farbstoffen, Mercurochrom, Silbersalzen und einem Extrakt aus blaugrünen Algen.

12. Verfahren nach einem der Ansprüche 1 bis 11, worin das Gewichtsverhältnis der Biozidlösung zu der ersten Latexschicht 0,05 bis 0,3 beträgt.

13. Verfahren nach einem der Ansprüche 1 bis 12, worin das Gewichtsverhältnis der Biozidlösung in der zweiten Schicht zu dem in der dritten Schicht aufgebrachten Latex zwischen 0,05 und 0,3 liegt.

14. Verfahren nach einem der Ansprüche 1 bis 13, worin das Biozid an die Oberfläche der Latexmembran gebunden ist und durch Diffusion in der Oberfläche der Latexmembran permanent fein verteilt ist.

## Revendications

1. Procédé de fabrication d'un article en latex ayant une barrière biocide, comprenant les étapes consistant à :
- appliquer sur une forme, un premier revêtement constitué essentiellement de latex liquide et exempt de biocide ;
- appliquer sur le premier revêtement de latex liquide, un second revêtement constitué essentiellement d'un biocide, efficace comme coagulant pour le latex liquide ; et
- appliquer audit second revêtement, un troisième revêtement constitué essentiellement d'un latex liquide et exempt de biocide.

2. Procédé selon la revendication 1, dans lequel le troisième revêtement est plus épais que le premier revêtement.

3. Procédé selon la revendication 1 ou 2, dans lequel le premier revêtement est lessivé avec de l'eau avant l'application du second revêtement.

4. Procédé selon l'une quelconque des revendications 1 à 3, dans lequel le second revêtement est séché avant l'application du troisième revêtement.

5. Procédé selon l'une quelconque des revendications 1 à 4, dans lequel ledit premier revêtement est appliqué en plongeant la forme dans du latex liquide.

6. Procédé selon l'une quelconque des revendications 1 à 5, dans lequel le second revêtement est appliqué sur le premier revêtement lorsque le latex dans le premier revêtement est à l'état de gel humide.

7. Procédé selon l'une quelconque des revendications 1 à 6, dans lequel le second revêtement est appliqué en pulvérisant une solution biocide.

8. Procédé selon l'une quelconque des revendications 1 à 6, dans lequel ledit second revêtement est appliqué en plongeant la forme dans une solution biocide.

9. Procédé selon l'une quelconque des revendications 1 à 8, dans lequel le biocide est appliqué comme une solution ayant une concentration de 0,10 à 5 pour-cent en poids.

10. Procédé selon l'une quelconque des revendications 1 à 9, dans lequel ledit troisième revêtement est appliqué en plongeant la forme dans une cuve de latex liquide.

11. Procédé selon l'une quelconque des revendications 1 à 10, dans lequel le biocide est choisi parmi la chlorhexidine, le sulfate de dextrane, le triclosan, le benzalkonium, la bétadine, le violet de gentiane, les colorants acriflavine et acridine, le mercurochrome, des sels d'argent, et un extrait d'algue bleue.

12. Procédé selon l'une quelconque des revendications 1 à 11, dans lequel le rapport en poids de la solution de biocide sur le premier revêtement de latex va de 0,05 à 0,3.

13. Procédé selon l'une quelconque des revendications 1 à 12, dans lequel le rapport en poids de la solution de biocide dans le second revêtement sur le latex appliqué dans le troisième revêtement est compris entre 0,05 et 0,3.

14. Procédé selon l'une quelconque des revendications 1 à 13, dans lequel le biocide est lié à la surface de la membrane en latex et diffuse de façon permanente dedans.
